# EUROPEAN PATENT SPECIFICATION

(11) **EP 2 614 787 B1**
(45) Date of publication and mention of the grant of the patent: **15.03.2017**
(21) Application number: 13151490.3
(22) Date of filing: 16.01.2013
(51) Int. Cl.: A61B 17/80, A61B 17/86

(54) **Multi-axial bone plate fixation**
Mehrachsige Knochenplattenfixierung
Fixation d'une plaque osseuse multi-axiale

(30) Priority: 16.01.2012 US 201261586853 P; 29.03.2012 US 201261617067 P; 03.05.2012 US 201261641900 P
(43) Date of publication of application: 17.07.2013
(73) Proprietor: Carbofix Orthopedics Ltd., 46724 Herzlia Pituach (IL)
(72) Inventor: Beyar, Mordechay, IL-30889 Caesarea (IL); Globerman, Oren, IL-46910 Kfar-Shmaryahu (IL)
(74) Representative: Nederlandsch Octrooibureau

(56) References cited:
- WO-A1-2011/042407
- WO-A2-2011/154891
- DE-A1- 4 343 117
- US-A1- 2004 073 218
- US-A1- 2006 009 771
- US-A1- 2010 234 847

## Description

### FIELD AND BACKGROUND OF THE INVENTION

The present invention, in some embodiments thereof, relates to bone plate fixation and, more particularly, but not exclusively, to a bone plate fixation component with a conical head for deforming a bone plate and/or being deformed thereby during attachment thereto.

Some background art is described below.

US Patent 6,206,881, publication number US6206881 B1 describes a bone plating system for the internal stabilization of a fractured bone. The system includes a bone plate having a plurality of conical plate holes tapering toward a bone contact surface and at least two bone screws for anchoring the bone plate to a bone. The screws have a conical head with a textured or structured lateral outside surface which is greater in hardness than the portion of the bone plate which is proximal to the plate holes.

US Patent Application 2010/0016858, publication number US20100016858 A1 describes apparatus and methods for tissue fixation using a bone plate configured to accommodate fixation devices (such as screws, for example) inserted at various angles. The bone plate is formed of a material softer than the material of the fixation devices, and is provided with fixation holes with rounded, curved interior walls (i.e., non-threaded holes) that allow the fixation devices to be inserted at various angles. The fixation holes have a diameter smaller than that of the fixation devices. As a result of the difference between the materials of the plate and of fixation devices, and of the difference between the diameters of the fixation holes and of the fixation devices, the fixation devices deform the interior walls of the fixation holes, therefore self-threading the holes at an advantageous angle.

Patent application PCT/EP2010/064781, published as WO 2011/042407 describes a device for synthesis of bone fractures, comprising a plate and screws for locking said plate. The plate is entirely made from biocompatible and radiolucent plastic material suitable to permit a radiographic control and an easy explantation of the plate and is provided with through holes for the screws fixing the plate on the bone. The screws comprise a threaded shank having an external diameter less than the diameter of said holes and a head having a thread with an external diameter greater than the diameter of said holes so as to permit insertion of the screws according to a plurality of directions and inclination with respect to the axis of the holes in the plate, said head being suitable to engage said holes by self-threading.

US publication number 2006/009771 A1 to Jorge et al. teaches "A bone fixation system including a plate and a set of fixation locking screw. The plate defines a set of locking screw holes each having an internal thread. Each respective locking screw has a head with an external structure that is adapted to self-tap into the internal thread of a given locking screw hole to secure the respective first-type fixation locking screw at an surgeon directed angle relative to the plate. This angle is defined during forcible insertion and rotation of the respective locking screw into the given screw hole. The system may also include unidirectional locking screws."

US publication number 2004/073218 A1 to Laurence teaches "A fastening apparatus includes a fastener and a fastener receiving member. The apparatus enables the fastener to be affixed to the fastener receiving member at a variable insertion angle selected by the user. The fastener includes an elongate section and an adjoining head section. Both the elongate section and the head section are threaded. The fastener receiving member includes one or more apertures through which one or more corresponding fasteners can be inserted. Each aperture includes a contact region formed or disposed on an inside surface defining the aperture. The contact region includes a porous matrix of protrusions or fiber metal having a density and strength sufficient to render contact region tappable by the thread of the head section of the fastener. The thread on the head section is driven into the contact region at the selected insertion angle. As a result, the thread of the head section taps into the material of the contact region such that the fastener is affixed to the fastener receiving member and maintained at the insertion angle."

US publication number 2010/234847 A1 to Impellizzeri et al. teaches "The self-locking osteosynthesis device includes a plate equipped with holes for the passage of fixation screws. The invention is characterized in that, at least in the areas defining the screw holes, the aforementioned plate is made from a material having mechanical properties such that the periphery of the holes can be self-tapped by means of tapping screws which can be used to fix the plate."

DE patent number DE4343117 teaches "a fixation system .... selectable and adjustable wheel angle between the bone plate and bone screw, which has a smaller footprint and is less expensive".

### SUMMARY OF THE INVENTION

The present invention relates to bone repair kit as claimed hereafter. Preferred embodiments of the invention are set forth in the dependent claims. Associated methods are also described herein to aid understanding of the invention, but these do not form part of the claimed invention.

There is provided in accordance with an exemplary embodiment of the disclosure a method not claimed of locking a fixation component into a bone plate formed of composite material, comprising deforming one or both of an aperture portion of said plate and a locking portion of said component that locks with said aperture portion, wherein said locking portion and said aperture portion have non-matching geometries.

In an exemplary embodiment of the invention, said aperture portion is generally cylindrical and wherein said locking portion is generally conical. Optionally or alternatively, said locking comprises locking by a combination of friction and interference-causing deformation of said aperture portion of said plate.

In an exemplary embodiment of the invention, said fixation component is a peg or a screw and wherein said locking portion is a head of said peg or screw. Optionally, a deformed section of said aperture component comprises the vicinity of a through hole in said plate. Optionally, said hole is smooth. Optionally or alternatively, said hole is cylindrical.

In an exemplary embodiment of the invention, said hole has a widening at a side away from a bone to which it is attached.

In an exemplary embodiment of the disclosure, the method not claimed, comprises inserting said fixation component at an angle to a central axis of said hole. Optionally, said angle is between 8 and 12 degrees. Optionally or alternatively, said angle is between 5 and 15 degrees.

In an exemplary embodiment of the invention, said insertion comprises locking said fixation component to said hole without causing any substantial wear in said hole.

In an exemplary embodiment of the invention, said insertion comprises using a fixation component with a head with a surface texture or structure harder than said hole. Optionally, said head is threaded.

In an exemplary embodiment of the invention, said deformation comprises deforming said locking portion and/or said aperture portion less than a mechanical failure of said portions.

In an exemplary embodiment of the invention, said deformation comprises not cutting said aperture portion by said locking portion.

There is provided in accordance with exemplary embodiments of the invention, a bone repair kit for allowing multi-axial insertion of fixation components through a plate, comprising:
(a) a composite material bone plate having at least one hole;
(b) at least one fixation component sized for locking in said hole and having a head, wherein said head has a geometry including at least one protrusion, which geometry does not match a geometry of said hole. Optionally, said at least one protrusion comprises a threading. Optionally, said threading is designed to not wear said bone plate hole. Optionally or alternatively, said threading is hard enough to distort a portion of the plate in a vicinity of said hole.

In an exemplary embodiment of the invention, said threading extends along at least 50% of a length of the head. Optionally or alternatively, said threading includes at least three circuits of said head.

In an exemplary embodiment of the invention, said fixation component has a threading along its body and wherein said threading of said head is shorter in radial extension than a threading of said body by a factor of at least 1.3, 2, 3, 4 or greater or intermediate factors.

In an exemplary embodiment of the invention, said fixation component has a threading along its body and wherein said threading of said body has a pitch larger than the pitch in the threading of said head.

In an exemplary embodiment of the invention, said fixation component has a threading along its body and wherein said threading has an intra-thread spacing different from that of a threading of said head. Optionally, said fixation component has a threading along its body and threading along its head wherein said head threading has more than one start.

In an exemplary embodiment of the invention, said hole is threadless.

In an exemplary embodiment of the invention, said hole does not include a conical cross-section.

In an exemplary embodiment of the invention, said hole has a cylindrical cross-section over at least 70% of a depth thereof.

In an exemplary embodiment of the invention, said hole has a widening at an opening thereof. Optionally, said widening also has a cylindrical cross-section.

In an exemplary embodiment of the invention, said head is conical. Optionally, said head has a tapering angle of between 5 and 20 degrees.

In an exemplary embodiment of the invention, said head is formed of a separate material from other parts of said fixation component.

There is provided in accordance with an exemplary embodiment of the invention, a bone fixation screw, comprising:
a body having a tip; and
a head having a surrounding and a base defining an attachment location for a rotating tool,
wherein said body is formed, at least mostly of a polymer or composite material and wherein said head is formed of said polymer or composite material and is covered with a hard layer of a material harder than said polymer or composite material, both at an outside and in said defined attachment location.

In an exemplary embodiment of the invention, the portion of said head formed of said polymer or composite material is conically shaped. Optionally or alternatively, the cover comprises an insert in said polymer or composite material. Optionally or alternatively, the cover is co-molded with said head.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

### BRIEF DESCRIPTION OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:
Fig. 1 shows a kit for bone fixation, in accordance with an exemplary embodiment of the invention;
Fig. 2 is a flowchart of a method of bone fixation using a kit according to Fig. 1, in accordance with an exemplary embodiment of the invention;
Fig. 3 shows the positioning of a bone plate on a fractured bone, in accordance with an exemplary embodiment of the invention;
Fig. 4 shows the selection of an angle for insertion of a fixation component into a bone, in accordance with an exemplary embodiment of the invention;
Fig. 5 shows the forming of a channel for the insertion of a fixation component into a bone, in accordance with an exemplary embodiment of the invention;
Fig. 6 shows the initial insertion of a fixation component into a bone, in accordance with an exemplary embodiment of the invention;
Fig. 7 shows a state of insertion of the fixation component of Fig. 6, where a head thereof contacts a bone plate, in accordance with an exemplary embodiment of the invention;
Figs. 8A-8B show a final state of insertion of the fixation component of Fig. 6, where a head thereof locks against a bone plate, in accordance with an exemplary embodiment of the invention;
Fig. 9 illustrates the deformation of a bone plate by a fixation component, in accordance with an exemplary embodiment of the invention;
Fig. 10A-10B illustrate a conical-head fixation component, in perspective and cross-sectional views, in accordance with an exemplary embodiment of the invention;
Figs. 11A-11C illustrate bone plates with multiple holes, in accordance with exemplary embodiments of the invention;
Fig. 12 is a cross-sectional view of a bone pate, illustrating various hole designs, in accordance with an exemplary embodiment of the invention;
Fig. 13A-13D illustrates a bone fixation screw with single start threading and a bone fixation screw with multi-start threading, in accordance with exemplary embodiments of the invention;
Figs. 14A-14C illustrate a bone fixation peg, in accordance with an exemplary embodiment of the invention;
Figs. 15A and 15B are a cross-sectional view of bone fixation components with a head covering of a harder material, in accordance with exemplary embodiments of the invention;
Fig. 15C shows a head hardening cover, in accordance with an exemplary embodiment of the invention;
Fig. 16A-16B illustrates alternative designs for a head of a bone fixation component, in accordance with an exemplary embodiment of the invention; and
Fig. 17A-17B illustrates a pairing of plate hole design and bone fixation design, in which the screw head is deformed by the plate, in accordance with an exemplary embodiment of the invention.

### DESCRIPTION OF SPECIFIC EMBODIMENTS OF THE INVENTION

The present invention, in some embodiments thereof, relates to bone plate fixation and, more particularly, but not exclusively, to a bone plate fixation component with a conical head for deforming a bone plate and/or being deformed thereby during attachment thereto.

### Overview

An aspect of some embodiments of the invention relates to locking a fixation component, such as a screw, to a bone plate. In an exemplary embodiment of the invention, the screw has a head which is deformed by and/or deforms that bone plate. In an exemplary embodiment of the invention, the bone plate has a hole which is generally cylindrical in form and the screw head is optionally conical. In an exemplary embodiment of the invention, the hole in the bone plate is unthreaded and is optionally formed by (e.g., during) compression molding or by drilling. Optionally, the hole in the plate includes a widened entrance and/or exit, optionally also cylindrical.

In an exemplary embodiment of the invention, the head of the screw has a threading which has multiple starts so as to have a lead which matches or is close in size to a lead on the screw body.

In an exemplary embodiment of the invention, the threading geometry is selected so as to reduce wear of the screw and/or bone plate and provide instead deformation of the screw and/or bone plate. In an exemplary embodiment of the invention, the deformation comprises forming of a thread channel in the bone plate. Optionally, at least 70%, 80%, 90% or more of the volume of the thread channel is provided by flowing or pushing away of bone plate material (or screw thread) by the screws and only at most part of the balance, if any, provided by degradation of the bone plate, for example, less than 30%, less than 20%, less than 10%, less than 5%.

In an exemplary embodiment of the invention, in use, the screw in inserted at a desired angle, for example within 15 degrees from the axis of the hole in the bone plate. When the screw head contacts the edges of the hole, the threading (or other geometry of head) interacts with the hole geometry. In an exemplary embodiment of the invention, the screw head is formed of or is covered with a material that is harder than that of the bone plate (or the bone plate hole is formed of or is covered with such a material), so that one is deformed by the other. Such deformation increases a friction and provides a mechanical locking of the screw head to the bone plate.

An aspect of some embodiments of the invention relates to a screw head design. In an exemplary embodiment of the invention, the screw head is designed to engage a hole in a plate over at least 70% of a length thereof, even if there is a mismatch between the head geometry and the hole geometry. In an exemplary embodiment of the invention, the screw head includes multiple threads, for example, 2, 3, 4, 5, 6 or more threads, as counted along the length of the head.

In an exemplary embodiment of the invention, the head is designed to include threading until within 20%, 10% or less of the proximal side of the head or, for example, within 1 mm, 0.5 mm, 0.2 mm, such that substantially all the head can engage the plate.

In an exemplary embodiment of the invention, when the screw is fully locked to its paired hole, there is no or substantially no protrusion above a surface of the plate, optionally even if the screw is at an angle of, for example, within 15 degrees of the hole axis.

In an exemplary embodiment of the invention, the screw geometry is selected so that when locked to the plate at a suitable torque, threads of the head deform or deform the plate so as to lock thereto. In an exemplary embodiment of the invention, a geometry of the head without its threads is selected so that it cannot all pass through the hole. In some embodiments, the head itself could pass the hole absent threads, however, the threads cause sufficient deformation and/or are deformed sufficiently to prevent such movement.

An aspect of some embodiments of the invention relates to a cover for a screw head which provides hardness to the screw head. Optionally, the cover also provided a recess for engaging of a screwdriver therein. Optionally, the screw has a conical head on which said cover is fitted.

In an exemplary embodiment of the invention, a bone implant device, such as a bone plate secured to the bone by bone screws, is composed of a composite material plate with at least one unthreaded screw aperture (hole) having a non-conical shape; and at least one screw having a threaded, conical shaped head which is made of material harder than that of the plate. In an exemplary embodiment of the invention, the conic screw head tapers toward the screw shank, and the diameter of the screw head at least along part of the screw head is larger than the plate aperture diameter, for example, due to the tapering of the head. In an exemplary embodiment of the invention, upon securing the plate to the bone, for example, by insertion of such a screw into the said plate hole and its rotation, the larger diameter conical screw head gradually pushes and compacts the interior wall of the non-conic plate hole so that the screw head is firmly locked to the plate. In an exemplary embodiment, the plate aperture has a substantial cylindrical shape. In an embodiment, other fixation device (such as a peg) may secure the plate to the bone (collectively referred to hereinafter as "screws").

In an exemplary embodiment, locking of the screw head to the plate is achieved with no self-tapping and optionally with self-threading by the screw head. Additionally and/or alternatively, locking of the screw head to the plate is achieved mainly by deforming of the interior wall of the plate.

In an exemplary embodiment of the invention, said design enables the surgeon to insert the screw into the plate hole and then to lock the screw to the plate, at more than one pre-designated direction/angle. This can be important in some situations, as the required insertion and locking angle of a screw may vary between cases and thus this feature allows addressing the need of each case individually, for more optimal positioning of the screw and effective fixation. A potential benefit of some designs is that having a conical shaped screw head enables the insertion of the screw in a wider range of directions/angle (compared, for example, to a cylindrical shape). In accordance with some embodiments, the screw may be inserted into the plate hole and locked to the plate in an angle, relative to the axis of the plate hole, of, for example zero (coaxial), or up to 10 degrees, or up to 15 degrees.

In an exemplary embodiment, following insertion and locking of the screw to the plate, the outside surface of the screw head does not protrude from the upper surface of the plate. In some embodiments, the outside surface of the screw head may slightly protrude from the upper surface of the plate.

In an exemplary embodiment, the screw head comprises connection mean for other tools, such as screwdriver. In some exemplary embodiments of the invention, said connection mean may be of any conventional shape, for example, an internally or externally threaded hexagon, Phillips head, axial crown, slotted, hexalobe (torx), etc.

In an embodiment of the invention, some of the plate holes or all of them are substantially cylindrical and unthreaded. In an embodiment of the invention, some of the screw heads or all of them are conical, threaded and made of material harder than the plate material.

In an exemplary embodiment of the invention, the composite material plate is made of carbon fiber-reinforced PEEK, and the fixation device (e.g., screw or peg) head is made of metal, such as titanium or titanium alloy (e.g., Ti-6Al-4V), or ceramics. In an exemplary embodiment, the entire screw/peg is made of material harder than the composite material plate, for example metal, such as titanium or titanium alloy or ceramics. In another embodiment, the screw/peg is made of composite material and is coated or partially coated and/or otherwise covered and/or includes an embedded component formed with material that increases the hardness of the coated component, for instance, metal or ceramic coating. In another embodiment, the screw/peg is made of composite material and its head comprises an insert made of harder material (for instance, metal or ceramics). In an embodiment, the insert is attached to the screw/peg head using adhesion means. Alternatively and/or additionally, the insert is molded into the screw using a geometric connection that prevents tear-off of the insert from the screw.

An aspect of some embodiments of the present disclosure relate to a method for locking a fixation device (e.g., screw) to a composite material bone plate, where the screw has a conical threaded head and the screw or screw head or screw head coating is made of material harder than that of the plate, and where the bone plate comprises a non-conical, optionally unthreaded screw hole with diameter smaller than the largest diameter of the conical screw head.

An aspect of some embodiments of the present disclosure relate to a method for insertion of a fixation device (e.g., screw) in various angles into a composite material bone plate and its locking to said plate in said various angles, where the screw has a conical threaded head and the screw or screw head or screw head coating is made of material harder than that of the plate, and where the bone plate comprises a non-conical, unthreaded screw hole with diameter smaller than the largest diameter of the conical screw head.

In an embodiment of the present invention, a non-conical (e.g., cylindrical) hole may be performed (drilled) in the bone plate intra-operatively at a desired location, and a screw with threaded conical shape head, having a largest diameter larger than the plate hole diameter and constructed from and/or coated by material harder than that of the plate, may be then introduced via the hole in a desired angle and further locked into the plate, optionally by compaction of the inner wall of the plate hole.

Before explaining at least one embodiment of the invention in detail, it is to be understood that the invention is not necessarily limited in its application to the details of construction and the arrangement of the components and/or methods set forth in the following description and/or illustrated in the drawings and/or the Examples. The invention is capable of other embodiments or of being practiced or carried out in various ways.

### Exemplary kit

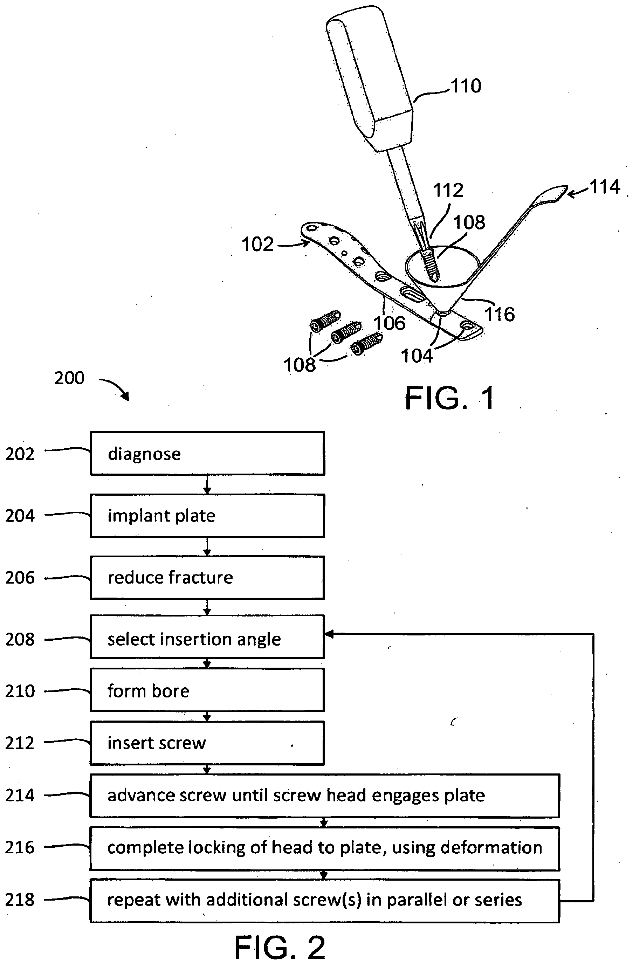

Fig. 1 shows a kit for bone fixation 100, in accordance with an exemplary embodiment of the invention. As shown, kit 100 includes a bone plate 102 having a body 106, for example, a plate for a distal Radius fracture, which plate includes one or more holes 104, which are optionally pre-drilled. Optionally, at least some of the holes are drilled when the plate is used (e.g., in operating room or in body).

In an exemplary embodiment of the invention, plate 102 is formed of a composite material and/or is otherwise radiolucent. Exemplary plates are described below. In some embodiments, a metal plate is provided, for example, a titanium plate, or a metal insert located in the holes of a plate formed of composite material.

Also shown are a plurality of bone fixation components 108, for example, screws. In an exemplary embodiment of the invention, the screws have a conical head, for example, as described below. In an exemplary embodiment of the invention, the screws are at least mostly formed of radiolucent materials and may include a relatively hard section possibly formed of radioopaque materials.

Optionally, a screwdriver 110, with a head 112 suitable for driving the screw, is provided.

Optionally, a screw guide 114 is provided, which includes a conically shaped head 116, which allows for aiming of screws within the angle of the cone. Optionally, the screw guide includes means to adjust the angle of the screw relative to the plate hole, for example a screw guide which can engage the hole at multiple angles or which is articulated.

### Exemplary method of bone fixation

Fig. 2 is a flowchart 200 of a method of multi-axial bone fixation, for example, using a kit according to Fig. 1, in accordance with an exemplary embodiment of the invention.
Figs. 2-8 illustrate exemplary stages of such a process, which may be repeated (218), at least in part, for insertion of additional fixation components.

### Channel formation and initial insertion

At 202, the patient is typically diagnosed as requiring a bone fixation plate, for example, based on x-ray imaging. It is noted that in some embodiments, a fixation component for a intramedullary nail is provided and which includes a thickening in its middle, for example, an ellipsoid section of the fixation component, so as to engage a bore in an intramedullary nail, at various angles thereof. Optionally, the bore is provided with an hourglass form or conical form to allow entrance of a fixation component thereto at various directions. Optionally, the "waist" of the hourglass has a significant length, for example, 1-5 mm, to engage the fixation component. Optionally, the above mentioned thickening, which may taper in one or two directions relative to the axis of the fixation component, has its maximal diameter at a point between 20% and 80%, or between 40% and 60% of the length of the fixation component.

At 204, a plate is implanted, for example, via an open incision along the bone, or a keyhole incision through which the plate is inserted and placed against the bone.

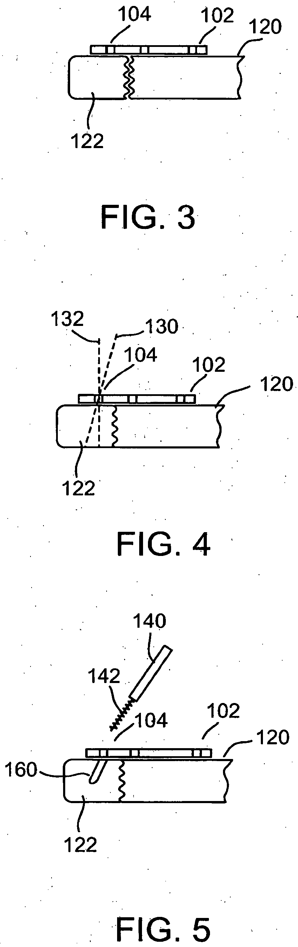

Fig. 3 shows the positioning of a bone plate 102 on a fractured bone 120 (with a fractured section 122), in accordance with an exemplary embodiment of the invention. Holes 104 of plate 102 are generally aligned with whole bone sections, but it is sometimes the case that the fracture lines of the bone are not perpendicular to the plate.

At 206 the fracture is optionally reduced, for example, using k-wires. In an exemplary embodiment of the invention, the k-wires are inserted through holes 104 and/or otherwise inserted after the plate is implanted. This can be done, for example, if the plate is radiolucent and fracture reduction is under x-ray.

Once the fracture is reduced and plate positioned, it may be determined that fixation components cannot be inserted perpendicular to the plate and still properly engage bone. In some cases this may be solved by changing the plate or by drilling new holes in the plate or by moving the plate. In an exemplary embodiment of the invention, however, fixation components are inserted into the bone at an angle so that they engage bone in a desired manner.

Fig. 4 shows the selection of an angle for insertion of a fixation component into a bone, in accordance with an exemplary embodiment of the invention.

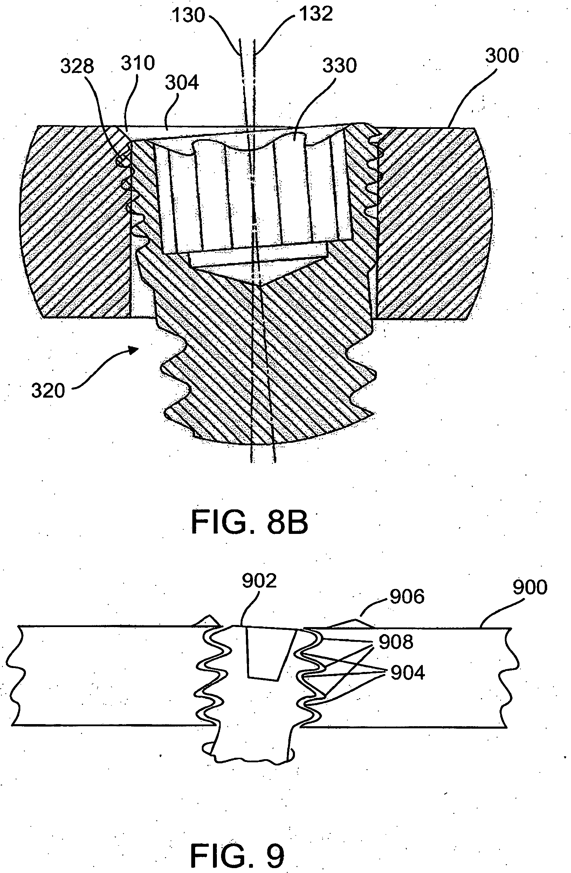

Screw 108 may be introduced into bone 120/122 and locked to plate 102 in various directions/angles relative to a plate aperture axis 132. As indicated above, the parameters of an individual case, such as patient bone anatomy, bone fracture type, desire to prevent contact between the screw and an intramedullary nail or other implant, in case of treating with a plate, bone that already treated by nail, hole location and/or other medical parameters, suggest an angle 130 at which the screw should be introduced for optimal fixation and/or effective healing. Optionally, this decision is made using x-ray imaging after the plate is inserted into the body. At least some of the embodiments described herein allow for variable angle insertion and locking of screw 108 to plate 102 (e.g., within a locking angle of up to 15 degrees between insertion axis 130 and plate hole axis 132). Fig. 8B shows a locking angle of about 8 degrees relative to the axis of the plate hole. While in some embodiments hole axis 132 is generally perpendicular to plate 102, in some plates this is not so.

Fig. 5 shows the forming of a channel 160 for the insertion of a fixation component 108 into bone 120/122, in accordance with an exemplary embodiment of the invention. Optionally, the hole is formed using a drill 140 with a drill burr 142. Optionally, a drill guide, not shown, is used to assist in aiming. Optionally, the screw guide 114 is used to control the drilling angle. In some embodiments the channel is provided by a k-wire. In an exemplary embodiment of the invention, the channel diameter is close to a diameter of the body of the screw (e.g., without the thread).

In an exemplary embodiment of the invention, the screw is cannulated, and can be guided over a k-wire, for example the cannulation having a lumen of, for example, 1.2 mm, or 1.5 mm to fit a matching k-wire, for example.

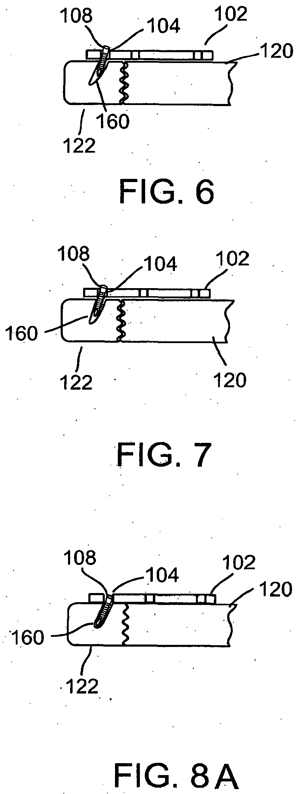

Fig. 6 shows the initial insertion of a fixation component such as screw 108 into bone 120/122 via channel 160, in accordance with an exemplary embodiment of the invention. In some embodiments, screw 108 is advanced along a k-wire

### Exemplary interaction between head and plate

Fig. 7 shows a state of insertion of the fixation component 108 of Fig. 6, where a head thereof contacts a bone plate, in accordance with an exemplary embodiment of the invention.

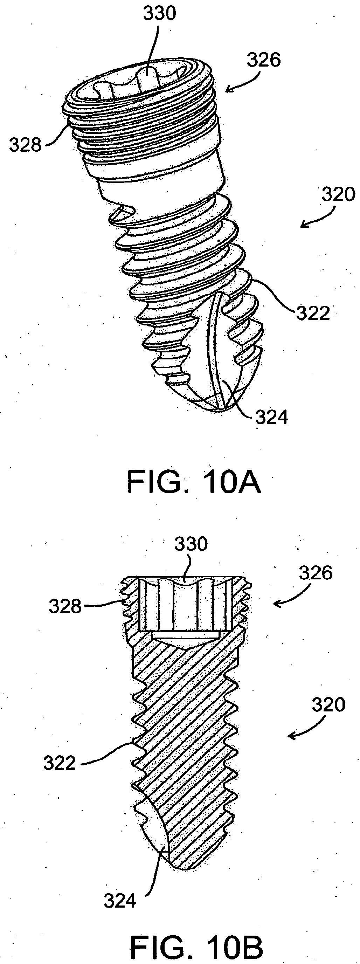

In an exemplary embodiment of the invention, following insertion of screw 108 into bone 120/122 through plate hole 104, screw 108 is rotated. Referring briefly to Fig. 10A, a screw 320, which is an exemplary embodiment of bone fixation component 108 may include a body with a thread 322 and a conical head 326 with a threading 328 and a dedicated recess for a screwdriver 330. In an exemplary embodiment of the invention, screw 320 is rotated using a screwdriver connected to a dedicated recess 330 at the screw upper surface, as described in Fig. 10A), and screw head threads 328 start to deform portions of the inner wall of plate hole 104, so that the threads 328 are forced into the said wall and locked to plate 102.

In an exemplary embodiment of the invention, this process includes little or no tapping (material removal) of the plate by the screw head threads 328. Removal of less than 0.2 mg of plate material per screw hole was shown in laboratory testing, for some embodiments of the invention. Optionally, a larger amount is allowed, for example, 5 mg, 2 mg, 1 mg or less. In some embodiments, even smaller amounts of material are removed.

Figs. 8A-8B show a final state of insertion of the fixation component 108, where a head thereof locks against a bone plate, in accordance with an exemplary embodiment of the invention.

Fig. 8A is a side cross-sectional view showing the head of fixation component 108 locked to and recessed in plate 102, or at least being flush with the surface of plate 102 (e.g., the upper surface of the screw does not protrude from the upper surface of the plate) and/or extending above less than, for example, 3 mm, 1 mm, 0.5 mm or 0.1 mm. Optionally, the head of fixation component 108 is rounded.

Fig. 8B illustrates in greater detail an exemplary desired locking between a plate aperture region 300 of a hole 304 and a screw 320. Plate hole 304 has a generally cylindrical configuration, and comprises an optional phase 310 at its upper portion. Screw 320 includes a head with conical configuration that comprises a thread 328.

In some embodiments of the invention the screw geometry and the hole geometry while not matching, are selected to have a desired relationship so as to provide one or more of locking, reduced wear, greater pull-out resistance and/or a desired range of angle.

Such selection is generated, for example, by providing a kit with plates and screw or by providing a set of screws with a table assigning certain screws to certain situations and/or desired effects. Optionally, the screws are color coded or otherwise marked to allow easy discrimination.

In some embodiments, the head has a main axis not aligned with the body of the screw. Optionally, the screwdriver slot is aligned with the axis of the body. Optionally or alternatively, the head is not rotationally symmetric (e.g., ignoring the thread).

In Fig. 8B, the thread 328 does not continue along the entire length of the screw head, but rather only along the upper portion of the screw head, so possibly only part of the plate material near hole 304 is deformed. This may reduce pull-out strength and/or require a greater deformation of plate material. In some cases, this may allow a greater range of angle of insertion to be provided. Nevertheless, thread along larger portion of the screw head or along the entire screw head are possible as well. In an exemplary embodiment of the invention, the threading is along at least 40%, 50%, 70%, 90%, or intermediate percentages of the length of the head.

In an exemplary embodiment of the invention, the outer diameter of the screw head thread 328 is larger, for example, by between 5% and 25%, for example, between 7 and 15%, at least at its proximal, widest portion, than the diameter of the plate hole 304. In an exemplary embodiment of the invention, the diameter of the head without its threads is smaller than a diameter of the hole in the plate.

Fig. 9 illustrates in greater detail the deformation of a bone plate 900 by a fixation component 902, in accordance with an exemplary embodiment of the invention.

As shown, the threads of screw 902 cause flowing of portions of plate 900. In one example some of the material flows away from the plate (original) surface, as indicated by 906. In some cases, different amounts of such flow are expected at the acute side and at the oblique side of the plate (acute being where the angle between screw 902 and plate 900 is acute and oblique being at the opposite side).

References 908 indicate areas where the threads pushed plate material generally radially away from the hole in plate 900. References 904 indicate areas where spacing between the threads or between the screw head and the walls of the plate hole allowed flow into the plate hole

Thread geometry and material properties for screw and plate may be selected to have various effects as described herein.

In an exemplary embodiment of the invention, the screw insertion creates a thread channel in the bone plate, at least 50% of which is optionally provided by pushing away of material by deformation.

In an exemplary embodiment of the invention, the volume between screw threads is filled with at least 10%, 20%, 30%, 40%, 50% or more material flowed in from the plate wall.

In an exemplary embodiment of the invention, use of flow deformation rather than wearing, reduces debris. Optionally, some cutting is allowed, especially if such cutting serves to separate material so as to allow it to better flow.

In some embodiments, the mechanism of forcing the screw head into the plate (rather than tapping) is enabled due to the differences in geometry, dimensions and/or material between the plate (and plate aperture) and the screw head and thread. Firm locking and resistance to pull out have been confirmed for some embodiments of the invention in laboratory testing.

In some exemplary embodiments of the invention, at least 30%, 50%, 70% or intermediate or smaller or greater percentages of the wall material in contact (e.g., to a depth of, for example, 0.1 mm or 0.3 mm or intermediate amounts) with the threads deforms more than 0.1 mm.

In some exemplary embodiments of the invention, a volume of the plate of at least 1 (or 3, 5, 7, 10 or intermediate volumes) cubic mm is deformed by flowing more than 0.1 (or 0.2, 0.5 or intermediate amounts) mm by a screw insertion.

In some exemplary embodiments of the invention, the screw head is of a harder material than the plate hole walls, so the plate deforms. In other embodiments, the plate is harder and the thread and/or screw head deform.

In an exemplary embodiment of the invention, most if not all (e.g., more than 90% by volume of deformed area) of the deformation is deformation without the formation of cracks or other failure modes of the plate (or screw).

Typical dimensions may be, for example, as follows: in distal fibula plate and distal volar radius plate, for instance, a typical diameter of hole in the plate shaft may be 3.7 - 4.1 mm, and the head diameter (e.g., including thread) of the complying screw may taper from 4.1 - 4.5 mm to 3.6 - 4.0 mm; a typical diameter of hole at the plate "head" may be 2.9 - 3.3 mm, and the head diameter of the complying screw may taper from 3.2 - 3.6 mm to 2.8 - 3.2 mm.

In an embodiment, the locking moment for screw having larger thread diameter of about 3.5 mm ranges from 0.7 - 1.6 N*m.The requested torque may depend, for example, on the tapering diameters, plate hole diameter, plate thickness and/or screw dimensions.

In some embodiments, the screw head taper (relative to its axis) is between 3 and 20 degrees, for example, between 5 and 15 degrees.

In some embodiments, the difference in taper angle between a screw head and the hole is between 1 and 15 degrees.

In some embodiments, the difference in angle between a screw axis and the hole axis is between 0 and 18 degrees.

While the embodiments described above focus on cylindrical holes and conical heads, also the holes may be conical. For example, the holes may have a tapering angle smaller than that of the heads or the holes may be reverse cones (tapering away from the bone). As noted below, the screw heads also need not be conical.

### Exemplary bone screw

Fig. 10A-10B illustrate a conical-head fixation component (a screw 320), in perspective and cross-sectional views, in accordance with an exemplary embodiment of the invention.

Screw 320 comprises a threaded shank 322 that is screwed into the treated bone. At its distal end, screw 320 may include a self-tapping tip 324, for example, as described in PCT publication WO2011/154891. At its proximal end, screw 320 comprises a head 326 of, for example, conical configuration, which tapers towards the screw shank 322 with an angle of, for example, between 5 and 15-20 degrees. As shown, screw head 326 includes a helical thread 328. Screw head 326 may comprise ring/s or other elements at its outside surface. For example, a circumferential ring/shoulder (optionally continuous) may be provided between the head and the body threadings and/or along the head and/or at a proximal side of the head. In an exemplary embodiment of the invention, no separate shoulder to prevent over insertion is provided.

In an exemplary embodiment of the invention, the upper surface of screw head 326 includes a connection means 330 for instrumentation such as an insertion/extraction screwdriver. Optionally, as shown in Fig. 10A, connection mean 330 has a hexalobe shape.

In an exemplary embodiment of the invention, screw 320 or at least screw head 326 is constructed from or covered with material which is harder than the bone plate material. For example, screw 320 or screw head 326 is made of metals such as titanium or titanium alloy (e.g., Ti-6Al-4V). Optionally, screw 320 or screw head 326 are made of ceramics. In some embodiments of the invention, screw 320 or screw head 326 are made of composite material such as carbon fibers-reinforced PEEK, and are coated with harder material/s (e.g., metal or ceramics, for example as described herein or as described in PCT publications WO2011/154891 and WO2010/082183).

The head of fixation element (or other locking portion thereof) need not be conical. In one example, the head tapers from its middle to both the proximal and distal ends thereof. Optionally, the taper angle is the same in both directions. Optionally, a ring is provided at the middle (or other location where the tapers meet, such as 60% of the length), which has a uniform diameter. In one example, the head is spherical or the shape of a truncated sphere or ellipsoid. Threading is optionally provided on one or both of the tapers and/or on the ring section, if any.

While the above description has focused on threading, other structural surface variations may be used, for example, protrusions or other geometries with radial variation on the head diameter e.g., with a radial variation of at least 0.1, 0.2, 0.3 or more mm, for example, in a plane perpendicular to the screw or fixation component longitudinal axis.

In one example, threading is interrupted, thus defining a series of helically arranged protrusions. Optionally, between 2 and 40, for example, between 4 and 20 such protrusions are provided. In an exemplary embodiment of the invention, the above number of protrusions each extend between 5 and 180 degrees of a circumference, for example, between 10 and 60 or 120 degrees.

In an exemplary embodiment of the invention, the leading end of such protrusions (or thread) is provided with a harder material, to ensure deformation. A trailing edge and/or body are also optionally so provided.

In an exemplary embodiment of the invention, the trailing edge of the protrusion or of a threaded section include an abrupt change in diameter (e.g., a step or missing section of the thread, e.g., with a length within which the change occur of less than 50%, 30%, 10% of the height of a thread), so as to resist unscrewing thereof, once the above described deformation occurs.

Optionally or alternatively, the threading ends with one or more protrusion with an abrupt leading edge, so as to provide increased resistance to further rotation.

In an exemplary embodiment of the invention, the threading or protrusions are provided by a metallic or ceramic or other hard insert provided in a mold in which the screw is formed, for example, a helix formed of a flat material may be used to define the threads.

In an exemplary embodiment of the invention, a thread has a height (radial extent) of 0.2 to 0.6 mm from its lowest to its highest part. Other sizes, larger or smaller may be provided as well.

In an exemplary embodiment of the invention, a thread base has a width of 0.3 to 0.8 mm. Other sizes, larger or smaller may be provided as well.

In an exemplary embodiment of the invention, a thread has a spacing between adjacent threads of 0.3 to 1 mm. Other sizes, larger or smaller may be provided as well.

In an exemplary embodiment of the invention, the peak of the thread is rounded, for example having a bending radius of 0.1 mm. Other sizes, larger or smaller may be provided as well.

While the above description has focused on screw heads with a generally circular cross-section, this need not be the case. In one example, the head has an elliptical cross-section. In another example, the head has different cross-sectional shapes and/or orientations of a same shape, at different axial positions thereof.

### Exemplary bone plate designs

Various bone plates may be used for the kit of Fig. 1. In one example, a standard, possibly threaded metal bone plate is used. In other example, a composite material bone plate, for example as described in PCT publication WO2011/154891 and/or using materials as described in PCT publication WO2010/082183, is used. Plates for various body parts may be provided, for example, to treat tibia, femur, Humors, fibula, Radius, ulna, Clavicle, Maxillofacial, Carnial, Spine and more. Also as described below, various hole designs may be used.

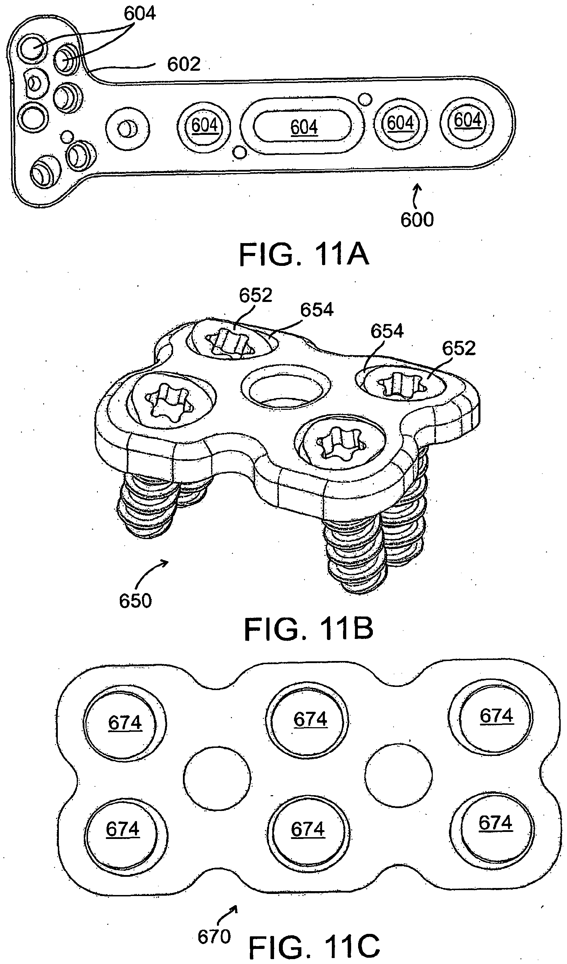

Fig. 11A is a top view of a bone plate 600 with multiple holes 604, including in a head 602 thereof, suitable for a distal Radius fracture in accordance with an exemplary embodiment of the invention. Optionally, a drill guide is used. Optionally, a drill guide is provided for use at angles other than the hole axis. As shown, some of the holes or of different sizes than others, a slot may also be provided and/or several holes have an axis not perpendicular to the plate surface.

Fig. 11B illustrates a bone plate 650 with multiple holes 654, suitable for cervical spine and showing optional screws 652 at various insertion angles locked thereto in accordance with an exemplary embodiment of the invention.

Fig. 11C is a top view of a different cervical spine plate 670, with multiple holes 674, which may be suitable, for example, for a two step fusion (attaching to three vertebra), in accordance with an exemplary embodiment of the invention. Optional holes which may be used for inserting bone cement or bone filler are also shown.

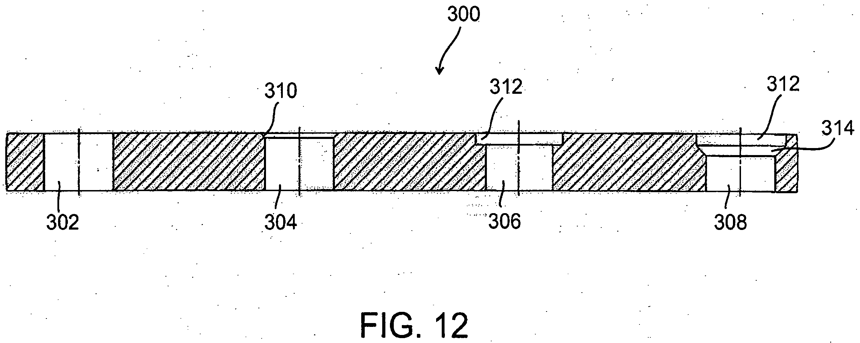

Fig. 12 is a cross-sectional view of a bone plate 300 illustrating various hole designs, in accordance with an exemplary embodiment of the invention.

In particular, in all of the illustrated embodiments, the hole is cylindrical over at least 80% or 90% of a length thereof and smooth and with a uniform round cross-section. This need not be the case for alternative embodiments which may, for example, has a surface geometry (such as threading), be conical, be slots or elliptical (e.g., with a ratio between diameters or cross-sectional dimensions of between 1.05 and 3 or 1.2 and 2) and/or otherwise be non-cylindrical over a length of, for example, between 10% and 90% of the hole, for example, between 20 and 50% thereof. In some cases, at least 1 mm, 2 mm, 3 mm or intermediate lengths of the hole are smooth and cylindrical.

A particular advantage which cylindrical holes may provide in some embodiments of the invention is that they may be simply formed by a peg in a mold, by boring (e.g., with a drill) and/or that such boring may be provided in an operating theater.

Referring back to Fig. 12, which schematically illustrates several exemplary designs indicated as screw holes 302, 304, 306, 308 having a substantially cylindrical shape, in accordance with some embodiments of the invention. Holes 302 -308 represent a few, generally cylindrical shaped holes, which differ somewhat from each other: plate hole 302 has a plain cylindrical aperture; hole 304 comprises at its upper section a small phase 310 (tapering towards the cylindrical hole), which may prevent sharp edges which might be undesirable in the body, for example, possibly causing tissue damage and/or implant degradation; hole 306 comprises at its upper section a "step" 312 with diameter larger than the rest of aperture, which step may be useful for accommodating a head of the screw; and hole 308 comprises at its upper section a "step" 312 and a phase 314. Optionally, multiple such steps are provided, optionally approximating a conical bore. For example, 2, 3, 4 or more such steps may be provided in a hole. It should be noted that such steps may also be formed by drilling, as may such a phase, as shown (by the leading edge o a conical drill). While not shown, such steps and/or phases may also or instead be provided at the bone side of the plate. In some cases, the holes have a bore and main axis that are not perpendicular to the plate, for example, at an angle of up to 15 or 30 degrees between the hole axis and perpendicular to plate surface. In some embodiments, the step is not symmetric and is designed (and/ or other features of the hole selected) to better support insertion at certain angles and/or orientations. Also not shown is the option of having a raised section or shoulder around all or part of the hole at the bone side and/or at the insertion side.

In some embodiments, plate holes for a particular plate are all of one design and/or dimensions. Alternatively, the different plate holes may be of different design and/or dimensions. For example, dimensions may be useful for using multiple screw sizes, e.g., simultaneously or as alternatives. For example, different designs and/or number may depended on, for example, one or more of the shape of the bone, the typical fracture shape and location and the strength of the bone in each location.

In an exemplary embodiment of the invention, during operation, for example, as described above, plate 300 is positioned over the fractured bone, and screws are introduced through its holes to secure the plate to the bone. In many situations it is desired that a firm locking of the screw/s to the plate will be achieved, avoiding the possibility of screw loosening over time. In addition, it is often desired that the screws may be introduced into the bone via the plate holes and then locked to the plate in more than one direction/angle, to comply with the different needs of different cases, and thus to allow for optimal fixation. Various embodiments as described herein provide for meeting such desires.

### Threading lead

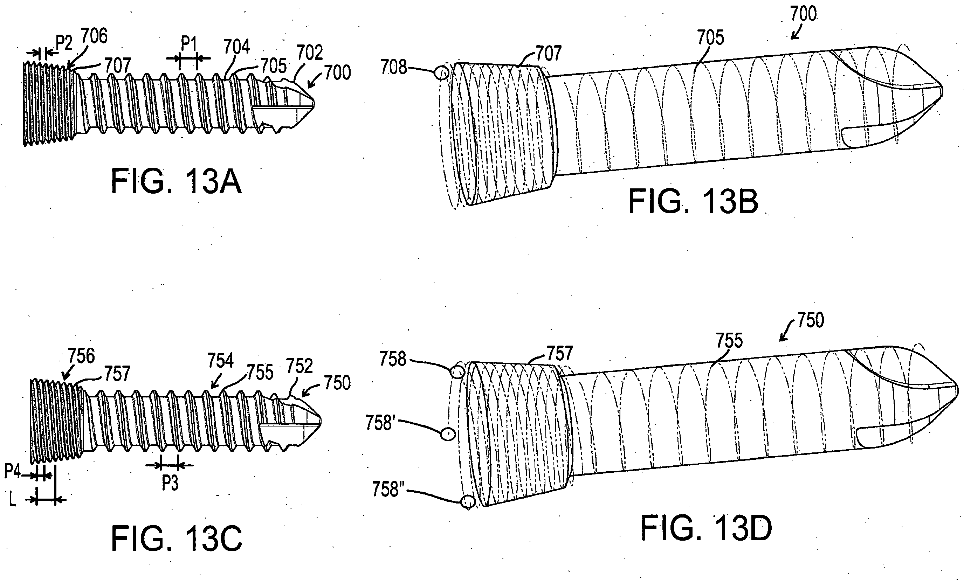

Figs. 13A-13B illustrate an exemplary bone screw 700 with a conical head 706, in accordance with an exemplary embodiment of the invention. As shown, screw 700 includes a body 704 with an optional threading 705 and/or a tip 702 which is optional self-tapping. Not shown, but optionally provided is a central core for riding on a k-wire and/or a radio-opaque marker(s) (e.g., in the distal tip and/or in the proximal end, optionally along the axis of the screw).

A potential problem with this design is that the pitch P2 of head threads 707 will general be smaller than a pitch P1 of body threads 705. This may be due, for example to their different functions - cutting into bone, vs. deforming a plate.

For example, P1 may be between 1.25 and 1.5 mm, while P2 might be about 0.5 mm. In an exemplary embodiment of the invention, the thread has a cross-section approximating a triangle with a base of width 0.5 and a height of 0.3. In an exemplary embodiment of the invention, the size of the base (relative to the height) is selected according to one or both of (a) the desired force of penetration of the thread into the plate and mechanical advantage due to screw rotation and (b) the number of desired penetrations. In an exemplary embodiment of the invention, it is desired to increase the number of penetrations so as to improve engagement and increase resistance to pullout. At a same time a depth of penetration is optionally selected, for example, at a depth which is sufficient to cause anchoring deformation and/or shearing, but not at a depth and/or geometry which might cause failure of the plate. In some embodiments, these tradeoffs leads to the provision of multiple low-height threads (e.g., as measured along length of head). Optionally, different thread sections have different radial heights.

Fig. 13B shows a perspective view with the threading shown only schematically and also showing that head threading 707 has only one start 708.

A potential shortcoming is that when the head engages the plate the plate will resist the forward motion dictated by thread 705 in the bone, possibly damaging the bone-thread connection or damaging the screw-plate connection. In some embodiments, the head threads are selected (e.g., radial height (e.g., smaller) and/or curve radius of peak (e.g., greater)) so that they can slip axially in the plate hole during tightening.

Figs. 13C and 13D show a multi-start threaded head which may be used to overcome the above problem and/or otherwise control a ration between the advance in bone and the advance in the plate.

As shown in Fig. 13C, (the reference numbers correspond to 13A with 50 added), a lead L is greater than a pitch P4 of head thread 757 and can be, for example, equal to a body thread pitch P3. It can also be made bigger or smaller therein. As shown in Fig. 13D, this change in lead (while maintaining a pitch suitable for engagement in the plate over multiple threads) is provided using a multi-start thread with, for example, three starts 758, 758' and 758".

In one example, pitch P3 is 1.5 mm, pitch P4 is 0.5 mm, but by providing three stats, a lead L of 1.5 is provided and which is equal to pitch P3 of body thread 755.

### Exemplary bone fixation peg

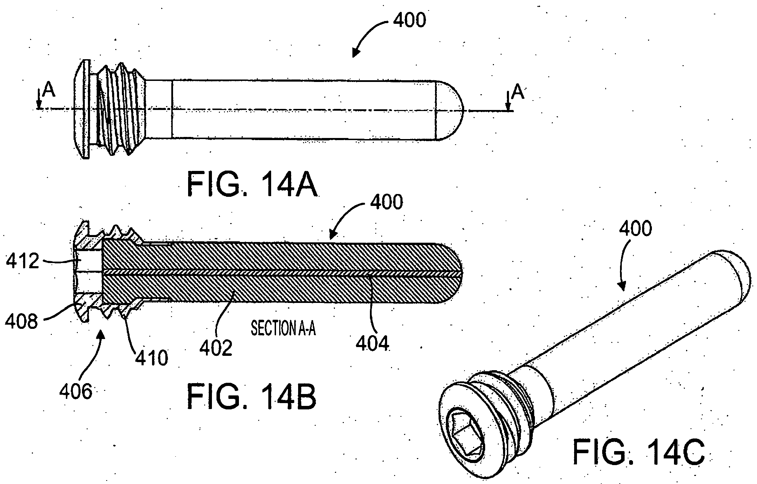

Figs. 14A-14C illustrate a bone fixation peg 400, in accordance with an exemplary embodiment of the invention. Such a peg need not have a body threading (or may have one only over less than, for example, 50%, 30%, 10% of its body.

Fig. 14B-14C are longitudinal cross section- and perspective views of the peg shown in Fig. 14A. In an exemplary embodiment of the invention, peg 400 is made of a composite material, such as PEEK reinforced with carbon fibers. Peg 400 includes a relatively smooth, non-threaded shank 402. In an exemplary embodiment of the invention, in order to visualize the radiolucent peg 400 under fluoroscopy, a radiopaque marker 404 is placed along the axis of the peg 400. Other forms or location of such marker/s are possible as well, for example, as described in PCT publication WO2011/15489. In some embodiments, marker 404 is replaced by a hollow lumen, for example, for riding on a k-wire.

In the embodiment shown, a peg head 406 is not conical. However, it may be conical, for example, as described above. Optionally, at least part of the peg head and/or threading thereof is of larger diameter than that of the plate hole.

In an exemplary embodiment of the invention, peg head 406, includes an insert 408 (or is made of such an insert or is covered with such an insert - insert being used a manufacturing term for molded items) which is made of material with greater hardness than the peg composite material. As shown, insert 408 comprises threads 410.

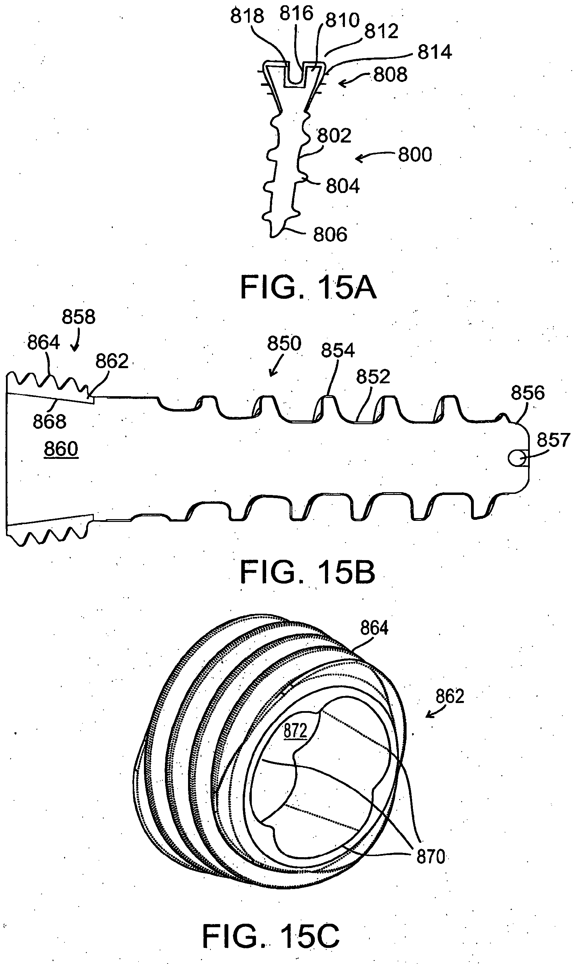

Peg head 406 optionally includes at its upper surface, optionally as part of insert 408, a connection means 412 to additional tools (e.g., a screwdriver for insertion). The insert 408 may be attached to the peg 400 using adhesion means. Alternatively and/or additionally, the insert may be molded into the peg 400 using a geometric connection that prevents tear-off of the insert from the screw. An example of such a mounting is shown in Fig. 15B, below.

According to some embodiments of the invention, such a peg may be introduced and locked to the plate in various angles (as described above for the screw), and upon its rotation, its thread compacts the interior wall of the plate hole to provide for a firm locking. Such a peg may be provided as part of a kit, for example, kit 100 of Fig. 1.

### Exemplary head hardeners

As noted herein, it may be desirable for the screw head to be harder than the plate material. The head design of Figs. 14A-14C may be used, as may other designs, for example, as shown in PCT publications WO2011/154891 and WO2010/082183.

Fig. 15A is a cross-sectional view of a bone fixation component 800 with a head covering 808 of a harder material, in accordance with an exemplary embodiment of the invention.

Component 800 may be, for example, a screw with a body 802, a body threading 804 (which may be hardened as well, not shown) and a tip 806, optionally self tapping.

In the example shown, head 808 is formed of an inner body 810 and an outer layer 812 which may be, for example, crimped, adhered or otherwise attached thereto or the head may be molded into layer 812 as an insert.

In an exemplary embodiment of the invention, inner body 810 has a minimal diameter greater than a minimal diameter of layer 812, to make inadvertent removal more difficult.

As shown, layer 812 optionally includes one or more threads or other protrusions 814 (optional, at least because bone plate may include threading or otherwise be non-circular). In an exemplary embodiment of the invention, layer 812 also includes a recess 816, matching a recess 818 in head 808, for an implantation and/or removal tool.

In an exemplary embodiment of the invention, layer 812 has a thickness of 0.1 to about 0.3 mm. Optionally, this thickness is selected to be sufficient so that threads do not deform at all or at least not more than desired, while deforming the plate.

Fig. 15B is a cross-sectional view of a screw 850 in accordance with an exemplary embodiment. As shown, screw 850 includes a body 852 with optional threads 854. Optionally, the threads are sized to not interfere with the hole in the plate for which it may be matched. Optionally, threads 854 are covered with a hard layer, such as metal.

A tip 856 of screw 850 is optionally self tapping. In an exemplary embodiment of the invention, a radio-opaque marker 857 is provided at a distal end thereof. Optionally or alternatively, a marker is provided at a proximal end thereof. Optionally, the marker is provided as an insert during molding of the screw. In an alternative embodiment, the marker is inserted after the screw is manufactured. In an exemplary embodiment of the invention, the radio-opaque marker is in the form of a ball, which may reduce artifacts, for example, in CT or MRI imaging. Such a radio-opaque marker may also be provided with other screw designs, for example, without head covers.

A head 858 of screw 850 has a body 860 optionally formed of a same material as and contiguous with body 852. In an exemplary embodiment of the invention, a hardening layer/cover 862 is provided and which is optionally co-molded (e.g., as an insert) with screw 850. As shown, cover 862 optionally includes threads 864. In other embodiments, also head 860 will include threads, covered by cover 862.

In the embodiment shown, cover 862 and body 860 contact along a conic surface 868. This angle of surface 868, for example, between 1 and 15 degrees, for example, between 3 and 5 degrees prevents pulling off of cover 862. Optionally, a step in body 860 and/or between body 860 and body 852, prevent movement in an opposite direction. A more complex contact geometry may be provided as well, for example, with non-monotonically varying diameter. Optionally, a radio-opaque marker is integrated into cover 862. Alternatively, for example to prevent weakening thereof, or additionally, a marker is provided embedded in body 860.

Fig. 15C is a perspective view of a head hardening cover 862, in accordance with an exemplary embodiment of the invention, as is optionally used for screw 850 and/or other screw designs. As shown, cover 862 defines a lumen 872 with is not of uniform radius. This may prevent separate rotation of cover 862 and screw 850. In other embodiments, such separate rotation is allowed and a circular cross-section may be provided. In an exemplary embodiment of the invention, the cross-section of lumen 872 defines three lobes 870 (a smaller or larger number may be provided in other embodiments).

In an exemplary embodiment of the invention, a recess for a screwdriver is formed in head body 860 (Fig. 15B). Alternatively, the screw driver may directly engage lobes 870. Alternatively, a separate insert may be provided to match the screw driver shape. So, in some embodiments, some of lumen 870 will not be covered by material of head 860.

In some embodiments (not shown) an upper portion of lumen 870 is shaped to match a desired screwdriver type, for example, hexalobe.

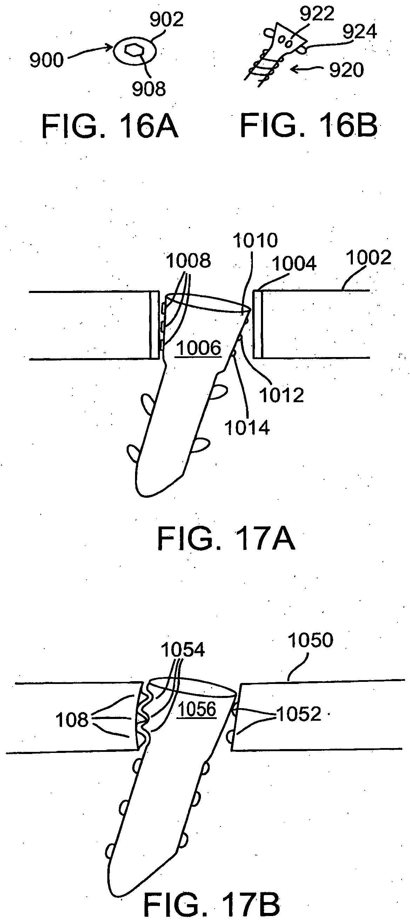

Figs. 16A and 16B illustrate some alternative designs for a head of a bone fixation component, in accordance with exemplary embodiments of the invention. For example, Fig. 16A shows a head 902 of a screw 900, which is elliptical in cross-section, at least at its top and has a tool/screwdriver recess 908.

Fig. 16B shows a screw 920 having a plurality of peg like protrusions 924 on the circumference of a head 922 thereof.

### Exemplary deformable screw head

As noted above, in some embodiments the plate deforms, in addition to or instead of screw deformation (or deformation of only threads thereof).

Fig. 17A shows a screw 1006 in a hole in a plate 1002. Plate 1002 is harder than head threads of screw 1006, or includes an insert or layer 1004 in the hole in the plate to provide such hardness (relative to screw head and/or threads). In one example, layer 1004 is made of metal. In another it is a polymer, for example, made of peek. Optionally, the threads on the head of screw 1006 are made of a softer material than the plate, for example, a suitable polymer.

As shown, thread sections 1008 at one side of the screw are substantially flattened due to compression, while only some of thread sections on an opposite side are so affected. For example, more proximal thread sections 1012 are compressed and more distal sections 1014 are not. It is noted that the plane of compression (e.g., defined by the edges of the compressed threads) need not be parallel to the plane of the surface of the screw head at adjacent locations and is, typically, dependent on the hole geometry.

Fig. 17B shows an alternative situation where a screw 1056, optionally with a conical (or other non-cylindrical) head, but optionally with no threads, is deformed (e.g., threads 1054 formed therein) by one or more threads or protrusions 1052 of a plate 1052. Protrusions 1052 may be provided using an insert of harder material (not shown) or by embedding harder material in the body of plate 1050. Alternatively, plate 1050 may e formed of such harder material.

For example, this means that a polymer or composite screw may be used with a standard threaded metal plate. Optionally, this also simplifies the task of thread formation in a composite screw.

### Exemplary manufacturing

Standard manufacturing techniques are optionally used for the embodiments described herein. It is noted, that providing a plate with non-threaded holes may not only contribute to the locking of the screw to the plate, as described before, but may also reduces the production costs of the plate and/or allow or make easier the formation of holes in an operating theater or prep-room or even in the body.

In an exemplary embodiment of the invention, the bone plate is made of composite material, for example PEEK reinforced with long carbon fibers. Optionally, the direction of the fibers vary in layers, for example, to be between 0 and 45 degrees in either direction from an axis of the plate, for example, to provide resistance to bending and/or other forces. Other angles, such as, for example, between 10 and 45 degrees or between 45 and 70 degrees or even up to 90 degrees or intermediate angles may be used, for example, depending on the part of the plate and/or part of body the plate is used in. Optionally, layers of different orientations are alternated.

In an exemplary embodiment of the invention, between 3 and 20 layers are provided, though other numbers of layers, such as 4, 7, or more may be provided. In an exemplary embodiment of the invention, the layers are provided by laying down tape of fibers in the desired orientations and adding PEEK or other filling polymer, followed by compression, if applicable.

In some cases, techniques such as described in PCT publications WO2011/154891 and WO2010/082183 are used.

Referring to Fig. 15B, the screw and head bodies 852 and 860 may be formed of a composite material of fibers and a polymer such as Carbon reinforced PEEK. Cover 862 may be formed of a metal such as Ti6Al4V. Main thread 854 may be formed of and/or covered with pure titanium or other metal and radio-opaque marker 857 is optionally formed of Tantalum.

Other biocompatible materials, such as metals, ceramics and polymers as well as cements and other materials may be used. Optionally, the materials are selected based on desired relative hardness, ability to deform without failing and/or reduced wearing of particles during and/or after implantation, for example, as described above.

### General

It is expected that during the life of a patent maturing from this application many relevant composite and polymer based materials will be developed and the scope of the term material is intended to include all such new technologies *a priori.*

As used herein the term "about" refers to ± 10%.

The terms "comprises", "comprising", "includes", "including", "having" and their conjugates mean "including but not limited to".

The term "consisting of" means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Although the invention has been described in conjunction with specific embodiments thereof, it is evident that many alternatives, modifications and variations will be apparent to those skilled in the art. Citation or identification of any reference in this application shall not be construed as an admission that such reference is available as prior art to the present invention. To the extent that section headings are used, they should not be construed as necessarily limiting.

## Claims

1. A bone repair kit for allowing multi-axial insertion of fixation components through a plate, comprising:
(a) a composite material bone plate (102) having at least one threadless hole (104);
(b) at least one fixation component (108) **characterized by** a conical head (326); said head including a thread (328);
**characterized in that** said hole is cylindrical over at least 80% of a length thereof and smooth with a uniform round cross-section, so that due to tapering of said conical head, a distal part of said head can be introduced via said hole in a desired angle, while a more proximal part of said head, including said thread, comprises a diameter larger than a diameter of said plate hole, so that said head is configured to lock to said plate by gradually deforming an interior wall of said hole during advancement of said fixation component within said hole.

2. A kit according to claim 1, wherein a volume of said plate of at least 1 cubic mm is deformed by flowing more than 0.1 mm by insertion of said fixation component into said hole.

3. A kit according to claim 1, wherein said threading is designed to not wear said bone plate hole.

4. A kit according to any of claims 1 -3, wherein said threading is hard enough to distort a portion of the plate in a vicinity of said hole.

5. A kit according to any of claims 1-4, wherein said threading extends along at least 50% of a length of the head.

6. A kit according to any of claims 1-5, wherein said threading includes at least three circuits of said head.

7. A kit according to any of claims 1-6, wherein said fixation component has a threading along its body and wherein said threading of said head is shorter in radial extension than a threading of said body by a factor of at least 2.

8. A kit according to claim 7, wherein said fixation component has a threading along its body and threading along its head wherein said head threading has more than one start.

9. A kit according to any of claims 1-5, wherein an outer diameter of said thread at a proximal, widest portion of the thread is between 5-25% larger than a diameter of said plate hole.

10. A kit according to any of claims 2-9, wherein said hole has a widening at an opening thereof.

11. A kit according to any of claims 1-10, wherein said head comprises a coating made of a material harder than the bone plate.

12. A kit according to claim 1, wherein said head has a tapering angle of between 5 and 20 degrees towards a shank of said fixation component.

13. A kit according to any of claims 1-12, wherein said head is formed of or includes a component of a separate and harder material from other parts of said fixation component.

14. A kit according to any of claims 1-13, wherein a body of said fixation component comprises a single start thread, and wherein a pitch of said head thread is at least two times smaller than a pitch of said body thread.

15. A kit according to any of claims 1-14, wherein said fixation component comprises composite material.

## Patentansprüche

1. Knochen-Reparaturkit zur Ermöglichung eines mehrachsigen Einführens von Befestigungskomponenten durch eine Platte, umfassend:
a) eine Verbundwerkstoff-Knochenplatte (102), die zumindest ein gewindeloses Loch (104) aufweist,
b) zumindest eine Befestigungskomponente (108), die charakterisiert ist durch einen konischen Kopf (326), der ein Gewinde (328) enthält,
**dadurch gekennzeichnet, dass** das Loch über zumindest 80% seiner Länge zylindrisch und glatt mit einem gleichmäßig runden Querschnitt ist, derart, dass aufgrund der Verjüngung des konischen Kopfes ein distaler Teil des betreffenden Kopfes durch das Loch in einem gewünschten Winkel eingeführt werden kann, während ein weiter proximal gelegener Teil des Kopfes, der das Gewinde enthält, einen größeren Durchmesser als einen Durchmesser des Plattenloches umfasst, derart, dass der betreffende Kopf gestaltet ist, um die Platte durch fortschreitendes Verformen einer Innenwand des Loches während des Vordringens der Befestigungskomponente innerhalb des Loches festzulegen.

2. Kit nach Anspruch 1, wobei ein Volumen der Platte von zumindest 1mm³ durch Fließen von mehr als 0,1mm durch Einführen der Befestigungskomponente in das genannte Loch verformt wird.

3. Kit nach Anspruch 1, wobei das Gewinde so ausgelegt ist, um das Knochenplattenloch nicht zu verschleißen.

4. Kit nach einem der Ansprüche 1 bis 3, wobei das Gewinde hart genug ist, um einen Teil der Platte in einer Nähe des Loches zu verziehen.

5. Kit nach einem der Ansprüche 1 bis 4, wobei das Gewinde längs zumindest 50% einer Länge des Kopfes verläuft.

6. Kit nach einem der Ansprüche 1 bis 5, wobei das Gewinde zumindest drei Kopf-Umläufe enthält.

7. Kit nach einem der Ansprüche 1 bis 6, wobei die Befestigungskomponente ein Gewinde längs ihres Körpers aufweist und wobei das Gewinde des Kopfes in radialer Längenausdehnung um einen Faktor von zumindest 2 kürzer ist als ein Gewinde des Körpers.

8. Kit nach Anspruch 7, wobei die Befestigungskomponente längs ihres Körpers ein Gewinde und längs ihres Kopfes ein Gewinde aufweist, wobei das Kopfgewinde mehr als einen Anfang aufweist.

9. Kit nach einem der Ansprüche 1 bis 5, wobei ein Außendurchmesser des Gewindes an einem proximalen, weitesten Teil des Gewindes zwischen 5-25% größer ist als ein Durchmesser des Plattenloches.

10. Kit nach einem der Ansprüche 2 bis 9, wobei das Loch an seiner einen Öffnung eine Aufweitung aufweist.

11. Kit nach einem der Ansprüche 1 bis 10, wobei der Kopf einen Überzug umfasst, der aus einem Material besteht, welches härter ist als die Knochenplatte.

12. Kit nach Anspruch 1, wobei der Kopf einen Verjüngungswinkel zwischen 5 und 20 Grad zu einem Schaft der Befestigungskomponente aufweist.

13. Kit nach einem der Ansprüche 1 bis 12, wobei der Kopf aus einer Komponente eines separaten und härteren Materials aus anderen Teilen der Befestigungskomponente gebildet ist oder diese enthält.

14. Kit nach einem der Ansprüche 1 bis 13, wobei ein Körper der Befestigungskomponente einen einzigen Gewindeanfang umfasst und wobei eine Steigung des Kopfgewindes zumindest zweimal kleiner ist als eine Steigung des Körpergewindes.

15. Kit nach einem der Ansprüche 1 bis 14, wobei die Befestigungskomponente einen Verbundwerkstoff umfasst.

## Revendications

1. Kit de réparation osseuse pour permettre l'insertion multiaxiale de composants de fixation à travers une plaque, comprenant :
(a) une plaque osseuse (102) en matériau composite comportant au moins un trou non-fileté (104) ;
(b) au moins un composant de fixation (108) **caractérisé par** une tête conique (326) ; ladite tête incluant un filetage (328) ;
**caractérisé en ce que** ledit trou est cylindrique sur au moins 80% de sa longueur et lisse avec une section transversale ronde uniforme, de sorte que, en raison de la conicité de ladite tête conique, une partie distale de ladite tête peut être introduite par l'intermédiaire dudit trou selon une l'angle désiré, tandis qu'une partie plus proximale de ladite tête, incluant ledit filetage, a un diamètre supérieur à un diamètre dudit trou de plaque, de sorte que ladite tête est configurée pour se verrouiller à ladite plaque par déformation progressive d'une paroi intérieure dudit trou pendant la progression dudit composant de fixation à l'intérieur dudit trou.

2. Kit selon la revendication 1, dans lequel un volume de ladite plaque d'au moins 1 millimètre cube est déformée par écoulement de plus de 0,1 mm par insertion dudit élément de fixation dans ledit trou.

3. Kit selon la revendication 1, dans lequel ledit filetage est conçu pour ne pas user ledit trou de la plaque osseuse.

4. Kit selon l'une quelconque des revendications 1 à 3, dans lequel ledit filetage est suffisamment dur pour déformer une partie de la plaque au voisinage dudit trou.

5. Kit selon l'une quelconque des revendications 1 à 4, dans lequel ledit filetage s'étend sur au moins 50% de la longueur de la tête.

6. Kit selon l'une quelconque des revendications 1 à 5, dans lequel ledit filetage comprend au moins trois circuits de ladite tête.

7. Kit selon l'une quelconque des revendications 1 à 6, dans lequel ledit composant de fixation comporte un filetage le long de son corps, et dans lequel ledit filetage de ladite tête est plus court dans le prolongement radial qu'un filetage dudit corps d'un facteur d'au moins 2.

8. Kit selon la revendication 7, dans lequel ledit composant de fixation a un filetage le long de son corps et un filetage le long de sa tête, ledit filetage de tête ayant plus d'un début.

9. Kit selon l'une quelconque des revendications 1 à 5, dans lequel le diamètre extérieur dudit filetage à une partie proximale la plus large du filetage est 5 à 25% plus grand que le diamètre dudit trou de plaque.

10. Kit selon l'une quelconque des revendications 2 à 9, dans lequel ledit trou présente un élargissement au niveau d'une ouverture de celui-ci.

11. Kit selon l'une quelconque des revendications 1 à 10, dans lequel ladite tête comprend un revêtement en matériau plus dur que celui de la plaque osseuse.

12. Kit selon la revendication 1, dans lequel ladite tête a un angle de conicité compris entre 5 et 20 degrés en direction d'une tige dudit élément de fixation.

13. Kit selon l'une quelconque des revendications 1 à 12, dans lequel ladite tête est formée d'un composant, ou comprend un composant, en matériau plus dur distinct par rapport aux autres parties dudit composant de fixation.

14. Kit selon l'une quelconque des revendications 1 à 13, dans lequel un corps dudit élément de fixation comprend un filetage de départ unique, et dans lequel un pas dudit filetage de tête est au moins deux fois plus petit qu'un pas dudit filetage de corps.

15. Kit selon l'une quelconque des revendications 1 à 14, dans lequel ledit composant de fixation comprend un matériau composite.
